# EUROPÄISCHE PATENTANMELDUNG

(11) **EP 3 124 010 A1**
(43) Veröffentlichungstag der Anmeldung: **01.02.2017**
(21) Anmeldenummer: 16189583.4
(22) Anmeldetag: 30.04.2014
(51) Int. Cl.: A61K 6/027, C03C 4/00, C03C 10/00, C03B 32/02, A61C 13/00, A61K 6/02

(54) **ROHLING UND VERFAHREN ZU SEINER HERSTELLUNG**

(30) Priorität: 03.05.2013 DE 102013104561
(62) Teilanmeldung aus: 14720971.2
(71) Anmelder: Dentsply Sirona Inc., York, PA 17401 (US)
(72) Erfinder: FECHER, Stefan, 63867 Johannesberg (DE); HÖRHOLD, Heiner, 63654 Büdingen (DE); SCHUSSER, Udo, 63755 Alzenau (DE); VOLLMANN, Markus, 63571 Gelnhausen (DE); KUTZNER, Martin, 63543 Neuberg (DE)
(74) Vertreter: Stoffregen, Hans-Herbert

(57) **Zusammenfassung**

Die Anmeldung bezieht sich auf einen Rohling zur Herstellung eines dentalen Formteils wie Inlay, Onlay, Krone oder Brücke. Damit aus dem Rohling problemlos ein dentales Formteil herausgearbeitet werden kann, insbesondere mit dünnen Wandstärken, ist vorgesehen, dass der Rohling aus einer Glaskeramik mit einer Dichte zwischen 30 % und 60 % der theoretischen Dichte und aus Glaskeramikpulveipartikel einer Korngrößenverteilung d₉₀ ≤ 80 µm besteht, wobei der Anteil an Lithiumsilikatkristallen 10 Vol.-% bis 90 Vol.-% beträgt.

## Beschreibung

Die Erfindung bezieht sich auf einen Rohling zur Herstellung eines dentalen Formteils, wie Inlay, Onlay, Krone oder Brücke, wobei der Rohling einen Anteil von mehr als 10 Vol.-% Lithiumsilikatkristallen enthält.

Ferner bezieht sich die Erfindung auf ein Verfahren zur Herstellung eines dentalen Formteils wie Inlay, Onlay, Krone oder Brücke. Auch nimmt die Erfindung Bezug auf ein monolithisches dentales Formteil.

Der WO 2012/080513 A1 ist ein Verfahren zur Herstellung dentaler Formteile aus porösem Glas zu entnehmen, das kristalline Anteile nicht aufweist. Die Dichte des Rohlings liegt zwischen 50 % und 95 % der theoretischen Dichte eines dichtgesinterten Rohlings. Aus einem diesbezüglichen Rohling werden monolithische dentale Formteile wie Kronen, Teilkronen, Brücken, In- oder Onlays mittels Fräsen hergestellt, wobei eine trockene Bearbeitung erfolgt.

Der WO 2011076422 A1 oder der WO 2012/059143 A1 sind Lithiumdisilikat-Glaskeramiken zu entnehmen, die zur Herstellung von dentalen Formteilen verwendet werden. Die entsprechenden Glaskeramiken sollen sich durch gute mechanische und optische Eigenschaften auszeichnen.

Aus der WO 2013/053865 A2 ist ein Rohling aus einer Lithiumsilikat-Glaskeramik bekannt, aus dem dentale Formteile hergestellt werden. Die Keramik enthält zwingend ein dreiwertiges Metalloxid aus der Gruppe Y₂O₃, Lä₂O₃, Yb₂-O₃, Bi₂O₃ und Mischungen dieser. Des Weiteren ist die Glaskeramik im Wesentlichen frei von K₂O und Na₂O.

Um Zahnersatz auf der Basis von Lithiumdisilikat-Glaskeramik herzustellen, ist es auch bekannt, zylinderförmige Pellets herzustellen und diese sodann in einer Muffel zu verpressen (EP 1 484 031 B1).

Die US 6 455 451 B1 beschreibt einen Rohling zur Herstellung eines dentalen Formteils, wobei der Rohling aus Glaskeramik einen Anteil von 35-60 Vol.-% an Lithiumsilikatkristallen enthält.

Aus der US 2012/0309607 A1 ist ein Rohling zur Herstellung eines dentalen Formteils bekannt, wobei der Rohling aus Glaskeramik einen Anteil von mehr als 10 Vol.-% an Lithiumsilikatkristallen enthält.

Ein Rohling zur Herstellung eines dentalen Formteils wird in der US 2008/120 994 A1 beschrieben. Der aus Glaskeramik bestehende Rohling weist einen Anteil von 60-80 Vol.-% an Lithiumsilikatkristallen auf.

Ein Rohling zur Herstellung eines dentalen Formteils mit einer Dichte zwischen 50 und 65 % der theoretischen Dichte ist aus der DE 10 2010 056 037 A1 bekannt.

Der vorliegenden Erfindung liegt die Aufgabe zu Grunde, einen Rohling herzustellen, aus dem problemlos ein dentales Formteil herausgearbeitet werden kann. Dabei sollen dünne Wandstärken erzielbar sein. Das Bearbeiten soll bei geringem Verschleiß der Werkzeuge durchgeführt werden können. Auch sollen sich die aus dem Rohling hergestellten dentalen Formteile durch gute mechanische Eigenschaften auszeichnen. Zumindest einer der zuvor angesprochenen Aspekte wird in Bezug auf einen Rohling zur Herstellung eines dentalen Formteils, wie Inlay, Onlay, Krone oder Brücke, dadurch gelöst, dass der Rohling aus einer Glaskeramik mit einer Dichte zwischen 30 % und 60 % der theoretischen Dichte des durchgesinterten Rohlings und aus Glaskeramikpulverpartikeln einer Korngrößenverteilung d₉₀ ≤ 80 µm, insbesondere einer Korngrößenverteilung d₅₀ zwischen 10 µm und 60 µm besteht, wobei der Anteil an Lithiumsilikatkristallen 10 Vol.-% bis 90 Vol.-% beträgt.

Ein entsprechender Rohling wird durch Fräsen bearbeitet, wobei sich überraschend gezeigt hat, dass der Werkzeugverschleiß gering ist, so dass teure z. B. mit Diamant besetzte Werkzeuge nicht erforderlich sind. Sind die aus einem entsprechenden Rohling herausgearbeiteten dentalen Formteile durchgesintert, zeigt sich ferner überraschenderweise eine hohe Festigkeit, wobei im Vergleich zu einem Zahnersatz, der aus einem dichtgesinterten Rohling herausgearbeitet wird, sich ein Festigkeitszuwachs zwischen 10 % und 50 % ergibt. Insbesondere besteht die Möglichkeit, dünnwandige Strukturen zu erzielen, die auch beim Durchsintern nicht zerstört werden, da die Rohlinge aufgrund des kristallinen Phasenanteils eine hinreichende Stabilität aufweisen. Hierdurch ist auch der Vorteil gegeben, dass beim Durchsintern unterstützende Hilfsmittel wie Stützstrukturen oder ein Ausfüllen von Kavitäten nicht erforderlich ist.

Insbesondere ist vorgesehen, dass der kristalline Anteil des Rohlings 30 Vol.-% bis 60 Vol.-% beträgt. Des Weiteren zeichnet sich der Rohling dadurch aus, dass dieser eine offene Porosität zwischen 50 Vol.-% und 60 Vol.-%, insbesondere zwischen 20 Vol.-% und 50 Vol.-% aufweist.

Aufgrund der gewählten Parametergrößen ist sichergestellt, dass beim mechanischen Bearbeiten, das insbesondere trocken erfolgt, eine hinreichende Oberflächenglattheit erzielbar ist, ohne dass es grundsätzlich nach dem Sintern einer Nachbearbeitung bedarf.

Bevorzugterweise sollte das Glaskeramikpulver eine Korngrößenverteilung d₅₀ ≤ 25 µm aufweisen.

Durch die Korngrößenverteilung sowie einer Porengröße von 0,1 µm bis 5 µm ergibt sich eine dichte Packung der Pulverpartikel in dem Rohling, so dass feine Kantenstrukturen problemlos ausgebildet werden können. Aus der herausgearbeiteten Oberfläche sind keine sichtbar herausgerissenen Körner feststellbar.

Um die geringe Porengröße im Bereich von 0,1 µm bis 5 µm zu erzielen, ist insbesondere vorgesehen, dass der Anteil an feinteiligen Glaspartikeln entsprechend hoch ist.

Insbesondere ist vorgesehen, dass der Rohling eine Scheiben-, Quader- oder Stabgeometrie aufweist, aus dem im gewünschten Umfang und in Abhängigkeit von der Größe des Rohlings eine oder mehrere dentale Formteile herausgearbeitet werden können. Dabei ist zum Einspannen des Rohlings in einer Fräsmaschine vorgesehen, dass von der Umfangsfläche diametral zum Schwerpunkt des Rohlings Mittel ausgehen, über die der Rohling fixiert werden kann. So besteht die Möglichkeit, aus dem Rohling Aussparungen herauszudrehen, in die sodann Fixiermittel aus der Bearbeitungsmaschine eingreifen. Alternativ können z. B. Adaptoren an der Umfangsfläche angebracht wie geklebt werden, die zum Halten in einer Bearbeitungsmaschine bestimmt sind. Es besteht jedoch auch die Möglichkeit, aus dem Rohling Vorsprünge herauszuarbeiten, die als Halterung dienen.

Insbesondere zeichnet sich die Erfindung durch einen Rohling aus Glaskeramikpulverpartikeln mit einer Zusammensetzung in Gew.-% aus mit:

| | |
|---|---|
| SiO2 | 46,0 - 72,0 |
| Li2O | 10,0 - 25,0 |
| ZrO2 | 6,5 - 14,0 |
| P2O5 | 1,0 - 10,0 |
| Al2O3 | 0,1 - 8,0 |
| K2O | 0,1 - 5,0 |
| CeO2 | 0,1 - 4,0 |
| B2O3 | 0,0 - 4,0 |
| Na2O | 0,0- 4,0 |
| Tb4O7 | 0,0-2,5 |

| | |
|---|---|
| sowie 0,0 bis 4,0 von zumindest einem Additiv | |

Bevorzugterweise ist die Zusammensetzung der Glaskeramikpulverpartikel des Rohlings in Gew.-%:

| | |
|---|---|
| SiO2 | 49,0 - 69,0 |
| Li2O | 11,5 - 24,0 |
| ZrO2 | 7,0 - 13,5 |
| P2O5 | 1,5 - 9,0 |
| Al2O3 | 0,2 - 7,5 |
| K2O | 0,2 - 4,5 |
| CeO2 | 0,2 - 3,5 |
| B2O3 | 0,0 - 3,5 |
| Na2O | 0,0 - 3,5 |
| Tb4O7 | 0,0 - 2,0 |

| | |
|---|---|
| sowie 0,0 bis 4,0 von zumindest einem Additiv. | |

Hervorzuheben ist eine Zusammensetzung der Glaskeramikpulverpartikel für den Rohling in Gew.%:

| | |
|---|---|
| SiO2 | 52,0 - 66,0 |
| Li2O | 12,0 - 22,5 |
| ZrO2 | 7,5 - 13,0 |
| P2O5 | 2,0 - 8,5 |
| Al2O3 | 0,3 - 7,0 |
| K2O | 0,3 - 4,0 |
| CeO2 | 0,3 - 3,5 |
| B2O3 | 0,0 - 3,0 |
| Na2O | 0,0 - 3,0 |
| Tb4O7 | 0,0 - 2,0 |

| | |
|---|---|
| sowie 0,0 bis 4,0 von zumindest einem Additiv. | |

Hervorzuheben ist des Weiteren eine Zusammensetzung der Glaskeramikpulverpartikel des Rohlings in Gew.-% mit:

| | |
|---|---|
| SiO2 | 55,0 - 63,0 |
| Li2O | 12,5 - 21,5 |
| ZrO2 | 8,0 - 12,0 |
| P2O5 | 2,5 - 8,0 |
| Al2O3 | 0,4 - 6,5 |
| K2O | 0,4 - 4,0 |
| CeO2 | 0,5 - 3,0 |
| B2O3 | 0,0 - 3,0 |
| Na2O | 0,0 - 3,0 |
| Tb4O7 | 0,0 - 2,0 |

| | |
|---|---|
| sowie 0,0 bis 4,0 von zumindest einem Additiv. | |

Besonders hervorzuheben ist eine Zusammensetzung der Glaskeramikpulverpartikel des Rohlings in Gew.-%:

| | |
|---|---|
| SiO2 | 58,0 - 60,0 |
| Li2O | 13,5 - 20,5 |
| ZrO2 | 8,5 - 11,5 |
| P2O5 | 3,0 - 7,5 |
| Al2O3 | 0,5 - 6,0 |
| K2O | 0,5 - 3,5 |
| CeO2 | 0,5 - 2,5 |
| B2O3 | 0,0 - 3,0 |
| Na2O | 0,0 - 3,0 |
| Tb4O7 | 0,0 - 1,5 |

| | |
|---|---|
| sowie 0,0 bis 4,0 von zumindest einem Additiv. | |

Das zumindest eine Additiv ist zumindest ein Additiv aus der Gruppe Farbpigment, Fluoreszenzmittel. Insbesondere ist vorgesehen, dass das Additiv zumindest ein Oxid aus der Gruppe BaO, CaO, MgO, MnO, Er2O3, Gd2O3, Pr6O11, Sm2O3, TiO2, V2O5, Y203 ist oder ein solches Oxid enthält.

Anzumerken ist, dass die Gesamtgewichtsprozente der Komponenten der Pulvermischung in jeder Zusammensetzung 100 Gew.-% betragen.

Die Erfindung zeichnet sich insbesondere auch aus durch ein Verfahren zur Herstellen eines dentalen Formteils, wie Inlay, Onlay, Krone oder Brücke, umfassend die Verfahrensschritte
- Herstellen einer Schmelze einer Zusammensetzung in Gew.-%:

| | |
|---|---|
| SiO2 | 46,0 - 72,0 |
| Li2O | 10,0 - 25,0 |
| ZrO2 | 6,5 - 14,0 |
| P2O5 | 1,0 - 10,0 |
| Al2O3 | 0,1 - 5,0 |
| K2O | 0,1 - 4,0 |
| CeO2 | 0,0 - 4,0 |
| B2O3 | 0,0 - 4,0 |
| Na2O | 0,0 - 2,5 |
| Tb4O7 | 0,0 - 2,5 |

| | |
|---|---|
| sowie 0,0 bis 4,0 von zumindest einem Additiv, | |

- Herstellen einer Glasfritte durch Verdüsen der Schmelze und Abschrecken in einem Medium,
- gegebenenfalls Erzeugen von Glaspulverpartikeln aus der Glasfritte mit einer Korngrößenverteilung d₉₀ ≤ 80 µm,
- Auskristallisieren von Lithiumsilikatkristallen mit einem Volumenanteil zwischen 10 % und 90 % durch eine erste Wärmebehandlung von entweder der Glasfritte oder den Glaspulverpartikeln in einem ersten Temperaturbereich bei einer Temperatur T₁ mit 500 °C ≤ T₁ ≤ 750 °C über eine Zeit t₁ mit 5 min ≤ t₁ ≤ 120 min,
- wobei dann, wenn die Glasfritte einer Wärmebehandlung unterzogen worden ist, Herstellen von Glaskeramikpartikeln mit einer Korngrößenverteilung d₉₀ ≤ 80 µm aus der wärmebehandelten Glasfritte erfolgt,
- Pressen der Glaskeramikpulverpartikel zu einem Rohling,
- Bearbeiten des Rohlings durch Fräsen zur Herstellung eines dem dentalen Formteil entsprechenden Vorformteils unter Berücksichtigung des Schwundverhaltens des Rohlings und
- Durchsintern des Vorformteils bei einer Temperatur T₂ mit 800 °C ≤ T₂ ≤ 1050 °C über eine Zeit t₂ mit 5 min ≤ t₂ ≤ 60 min.

Erfindungsgemäß besteht die Möglichkeit, dass entweder die Glasfritte, ohne dass diese zuvor gemahlen wird, um Glaspulverpartikel zu erhalten, einer Wärmebehandlung zur Bildung von Lithiumsilikatkristallen unterzogen wird, oder aber die Fritte zunächst gemahlen wird, also Glaspulveipartikel erzeugt werden, um sodann die Wärmebehandlung durchzuführen, so dass sodann Glaskeramikpulverpartikel vorliegen. Das Merkmal "Pressen der Glaskeramikpulverpartikel zu einem Rohling" umfasst folglich die nach den Verfahrensalternativen hergestellten Glaskeramikpulverpartikel ein.

Dabei besteht die Möglichkeit, die erste Wärmebehandlung zweistufig innerhalb des ersten Temperaturbereichs durchzuführen.

Insbesondere weist die Schmelze eine Zusammensetzung in Gew.-% auf:

| | |
|---|---|
| SiO2 | 49,0 - 69,0 |
| Li2O | 11,5 - 24,0 |
| ZiO2 | 7,0 - 13,5 |
| P2O5 | 1,5 - 9,0 |
| Al2O3 | 0,2 - 7,5 |
| K2O | 0,2 - 4,5 |
| CeO2 | 0,2 - 3,5 |
| B2O3 | 0,0 - 3,5 |
| Na2O | 0,0 - 3,5 |
| Tb4O7 | 0,0 - 2,0 |

| | |
|---|---|
| sowie 0,0 bis 4,0 von zumindest einem Additiv. | |

Bevorzugterweise besteht die Schmelze aus einer Zusammensetzung in Gew.-%:

| | |
|---|---|
| SiO2 | 52,0 - 66,0 |
| Li2O | 12,0 - 22,5 |
| ZiO2 | 7,5 - 13,0 |
| P2O5 | 2,0 - 8,5 |
| Al2O3 | 0,3 - 7,0 |
| K2O | 0,3 - 4,0 |
| CeO2 | 0,3 - 3,5 |
| B2O3 | 0,0 - 3,0 |
| Na2O | 0,0 - 3,0 |
| Tb4O7 | 0,0 - 2,0 |

| | |
|---|---|
| sowie 0,0 bis 4,0 von zumindest einem Additiv. | |

Besonders hervorzuheben ist eine Zusammensetzung der Schmelze mit einer Zusammensetzung in Gew.-%:

| | |
|---|---|
| SiO2 | 55,0 - 63,0 |
| Li2O | 12,5 - 21,5 |
| ZiO2 | 8,0 - 12,0 |
| P2O5 | 2,5 - 8,0 |
| Al2O3 | 0,4 - 6,5 |
| K2O | 0,4 - 4,0 |
| CeO2 | 0,5 - 3,0 |
| B2O3 | 0,0 - 3,0 |
| Na2O | 0,0 - 3,0 |
| Tb4O7 | 0,0 - 2,0 |

| | |
|---|---|
| sowie 0,0 bis 4,0 von zumindest einem Additiv. | |

Bevorzugterweise ist vorgesehen, dass die Schmelze eine Zusammensetzung in Gew.-% aufweist:

| | |
|---|---|
| SiO2 | 58,0 - 60,0 |
| Li2O | 13,5 - 20,5 |
| ZrO2 | 8,5 - 11,5 |
| P2O5 | 3,0 - 7,5 |
| Al2O3 | 0,5 - 6,0 |
| K2O | 0,5 - 3,5 |
| CeO2 | 0,5 - 2,5 |
| B2O3 | 0,0 - 3,0 |
| Na2O | 0,0 - 3,0 |
| Tb4O7 | 0,0 - 1,5 |

| | |
|---|---|
| sowie 0,0 bis 4,0 von zumindest einem Additiv. | |

Das zumindest eine Additiv ist zumindest ein Additiv aus der Gruppe Farbpigment, Fluoreszenzmittel. Insbesondere ist vorgesehen, dass das Additiv zumindest ein Oxid aus der Gruppe BaO, CaO, MgO, MnO, Er2O3, Gd2O3, Pr6O11, Sm2O3, TiO2, V2O5, Y203 ist oder ein solches Oxid enthält.

Nach einem weiteren Vorschlag ist vorgesehen, dass der Rohling nach der ersten Wärmebehandlung und vor der mechanischen Bearbeitung getempert wird bei einer Temperatur T₃ mit 750 °C ≤ T₃ ≤ 900 °C über eine Zeit t₃ mit 5 min ≤ t₃ ≤ 30 min.

Durch die diesbezüglichen Wärmebehandlungsschritte wird sichergestellt, dass das Glaspulver im erforderlichen Umfang zu Lithiumsilikatkristallen kristallisiert, gleichzeitig eine geringe Porengröße und damit dichte Kornpackung ergibt, so dass eine problemlose mechanische Bearbeitung u. a. zur Erzielung filigraner Bereiche ermöglicht wird.

Die Kristallphase Lithiumsilikat schließt dabei Lithiummetasilikat und insbesondere Lithiumdisilikat ein.

Insbesondere ist vorgesehen, dass zur Herstellung eines Rohling in Scheibengeometrie die Glaskeramikpulverpartikel zunächst axial verpresst und sodann nach Einbringen in eine Umhüllende wie Beutel aus innenseitig beschichtetem Polyethylen isostatisch nachverdichtet werden, wobei das Nachverdichten insbesondere bei einem Druck pn mit 250 MPa ≤ pn ≤ 350 MPa über eine Zeit t₄ mit 5 sec ≤ t₄ ≤ 30 sec, insbesondere 5 sec ≤ t₄ ≤ 15 sec durchgeführt wird.

Zur Herstellung eines Rohlings in Quadergeometrie sieht die Erfindung vor, dass die Glaskeramikpulverpartikel sukzessiv und insbesondere kontinuierlich mit ansteigendem Druck über eine Zeit t₅ axial verpresst werden, wobei maximaler Druck p₅ beträgt 50 MPa ≤ p₅ ≤ 400 MPa, insbesondere 100 MPa ≤ p₅ ≤ 200 MPa. Der Zeitraum, innerhalb dem der Druckanstieg erfolgt, beträgt 10 sec ≤ t5 ≤ 20 sec.

Zur Herstellung eines Rohlings stabförmiger, insbesondere zylindrischer Geometrie, ist vorgesehen, dass das Glaskeramikpulver in eine schlauchförmige Pressform insbesondere aus Polyurethan eingebracht und sodann quasi isostatisch verpresst wird. Dabei sollten insbesondere nachstehende Presszeiten und Parameter berücksichtigt werden. Es wird zunächst ein langsamer Druckanstieg bevorzugt, um das eingefüllte Glaskeramikpulver gleichmäßig in der Form zu verteilen. Danach kann der Druck rasch auf seinen Maximalwert erhöht werden. Nach Erreichung des maximalen Drucks wird dieser für die Haltezeit konstant gehalten. Es folgt eine Phase der schnellen Druckentlastung, bei der der Druck bis auf 10% des Maximalwerts abgebaut wird. Der vollständige Abbau des Überdrucks erfolgt danach langsam, um Rissbildungen im Glaskeramikrohling zu vermeiden.

Zum mechanischen Bearbeiten, wobei eine trockene Bearbeitung möglich ist, ist insbesondere vorgesehen, dass zunächst eine Grobbearbeitung und sodann eine Feinbearbeitung erfolgt.

Bei der Grobbearbeitung sind bevorzugte Fräsparameter:

| | |
|---|---|
| Fräserdurchmesser: | 2 bis 5 mm, besonders 2 bis 3 mm |
| Vorschub: | 500 bis 4000 mm/min, besonders 2000 bis 3000 mm/min |
| Seitliche Zustellung ae: | 0,2 bis 3 mm, besonders 1 mm bis 2 mm |
| Tiefenzustellung ap: | 0,1 bis 2 mm, besonders 0,5 mm bis 1 mm |
| Fräserdrehzahl: | 10.000 bis 50.000 1/min, besonders 10.000 bis 20.000 1/min. |

Als Fräser sind Hartmetall-Fräser zu bevorzugen.

Bezüglich der Feinbearbeitung sollten nachstehende Fräsparameter beachtet werden:

| | |
|---|---|
| Fräserdurchmesser: | 0,3 bis 1,5 mm, besonders 0,5 bis 1,0 mm |
| Vorschub: | 300 bis 2000 mm/min, besonders 800 bis 1500 mm/min |
| Seitliche Zustellung ae: | 0,2 bis 0,6 mm, besonders 0,1 mm bis 0,2 mm |
| Tiefenzustellung ap: | 0,05 bis 0,3 mm, besonders 0,1 mm bis 0,15 mm |
| Fräserdrehzahl: | 20.000 bis 60.000 1/min, besonders 25.000 bis 35.000 1/min. |

Auch hierbei sind Hartmetall-Fräser zu bevorzugen.

Besonders gute Bearbeitungsergebnisse zeigen sich dann, wenn als Fräser ein Kugelradiusfräser aus Hartmetall verwendet wird, wobei sich der Kugelradiusfräser durch nachstehende Schneidenwinkel auszeichnen sollte:

| | |
|---|---|
| Spanwinkel: | 0° bis -13°, besonders -9° bis -11° |
| Freiwinkel: | 0° bis 15°, besonders 11° bis 13° |
| Keilwinkel: | ergibt sich 90° minus Freiwinkel minus Spanwinkel. |

Ist es dem Grunde nach nicht erforderlich, dass vor dem Verpressen der Glaskeramikpartikel ein Bindemittel zugegeben wird, so wird die Erfindung nicht verlassen, wenn entsprechende Bindemittel wie z. B. Celluloseether mit einem Gewichtsanteil von bis zu 5 % zugegeben werden.

Insbesondere hat sich jedoch als Vorteil erwiesen, wenn der Rohling, also dessen Glaskeramikpartikel nach dem Verpressen in Kieselsäure oder Alkalisilikatlösung (Wasserglas) getaucht werden und nach dem Trocknen mechanisch bearbeitet werden. Hierdurch bilden sich zwischen den Glaspartikeln SiO-Brücken aus, durch die die Festigkeit gesteigert und somit die anschließende mechanische Bearbeitung, die ein CAD-/CAM-Bearbeiten einschließt, erleichtert wird. Beim Durchsintern der bearbeiteten Formteile diffundiert das freie SiO2 in die Glaskeramik, wodurch eine Festigkeitserhöhung erzielbar ist.

Die Erfindung zeichnet sich auch durch ein monolithisches dentales Formteil aus, das unter Verwendung eines erfindungsgemäßen Rohlings hergestellt ist. Insbesondere kann das monolithische dentale Formteil eine Krone sein oder umfassen mit einem Kronenrand einer Dicke D_{R} mit 0,05 mm ≤ D_{R} ≤ 0,4 mm, insbesondere 0,1 mm ≤ D_{R} ≤ 0,2 mm. Die Dicke des Kronenrands erstreckt sich dabei vom Stirnrand aus in einem Abstand von 2 bis 4 mm zu diesem.

Des Weiteren zeichnet sich das monolithische Formteil durch einen Wärmeausdehnungskoeffizienten, gemessen nach ISO6872 dadurch aus, dass dieser kleiner als 12,5 x 10⁻⁶ 1/K ist, vorzugsweise zwischen 9,5 x 10⁻⁶ 1/K und 11,5 x 10⁻⁶ 1/K liegt.

Um die Glaskeramikpartikel zu verpressen, wird insbesondere ein Druck zwischen 50 MPa und 400 MPa, insbesondere zwischen 100 MPa und 200 MPa gewählt. Die Temperatur beim Vorsintern des kompaktierten Glaspulvers, also des Rohlings in Form des gepressten Glaskörpers, sollte im Bereich zwischen 500 °C und 950 °C, vorzugsweise zwischen 600 °C und 700 °C liegen.

Die Außengeometrie des gepressten Glaskeramikkörpers kann scheiben- bzw. plattenförmig oder stangen- wie zylinderförmig sein, wobei die Querschnittsgeometrie frei wählbar ist. Der Volumeninhalt der Rohlinge kann zwischen 1 cm³ und 160 cm³ liegen.

Nach dem mechanischen Bearbeiten der aus der kristallinen porösen Glaskeramik bestehenden Rohlinge, wobei bevorzugterweise eine Fräsbearbeitung ohne Kühlung erfolgt, werden die herausgearbeiteten dentalen Arbeiten in einem geeigneten Sinterofen unter Berücksichtigung eines geeigneten Temperatur-/Zeitzyklusses durchgesintert. Das Durchsintern kann dabei in einem Temperaturbereich zwischen 700 °C und 1100 °C, vorzugsweise im Bereich zwischen 850 °C und 950 °C durchgeführt werden. Die Gesamtzykluszeit beträgt weniger als 2 h, vorzugsweise weniger als 1 h. Aufgrund des kristallinen Anteils ist dabei nicht erforderlich, dass das Vorformteil gestützt wird. Vielmehr kann das Vorformteil z. B. auf einer Al₂O₃-Brennwatte im Sinterofen gelagert werden.

Als bevorzugter Temperatur-/Zeitzyklus ist anzugeben: Bereitschaftstemperatur 500 °C, Anstiegsrate 50°C/min bis 90°C/min auf 850 bis 900 °C, Haltezeit 1 bis 5 min, dann langsam abkühlen. Für die Abkühlung ist vorzugsweise die langsamste Kühlstufe zu wählen.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus den nachfolgenden Ausführungsbeispielen.

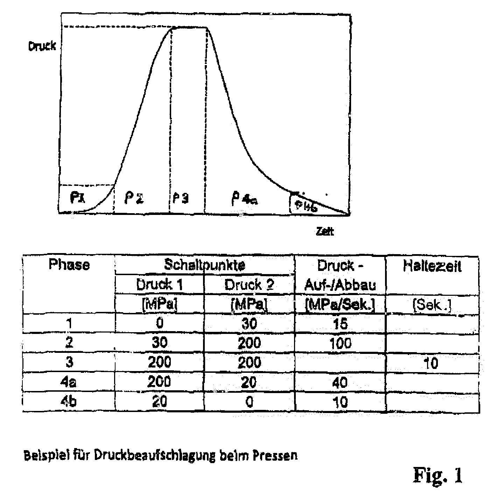

Die einzige Figur zeigt einen Verlauf des Drucks über die Zeit beim Verpressen eines Rohlings.

Um ein dentales Formteil herzustellen, wird erfindungsgemäß ein Rohling benutzt, der aus verpresstem Glaskeramikpulver besteht. Um das Glaskeramikpulver zur Verfügung zu stellen, wird zunächst ein Pulver aufgeschmolzen und aus der Schmelze eine Glasfritte hergestellt, die nachstehend bevorzugte Zusammensetzungen aufweisen kann:

| | |
|---|---|
| SiO2 | 49,0 - 69,0 |
| Li2O | 11,5-24,0 |
| ZrO2 | 7,0 - 13,5 |
| P2O5 | 1,5 - 9,0 |
| Al2O3 | 0,2 - 7,5 |
| K2O | 0,2 - 4,5 |
| CeO2 | 0,2 - 3,5 |
| B2O3 | 0,0 - 3,5 |
| Na2O | 0,0 - 3,5 |
| Tb4O7 | 0,0 - 2,0 |

| | |
|---|---|
| sowie 0,0 bis 4,0 von zumindest einem Additiv. | |

Insbesondere ist vorgesehen, dass die Glasschmelze eine Zusammensetzung in Gew.-% aufweist:

| | |
|---|---|
| SiO2 | 49,0 - 69,0 |
| Li2O | 11,5 - 24,0 |
| ZiO2 | 7,0 - 13,5 |
| P2O5 | 1,5 - 9,0 |
| Al2O3 | 0,2 - 7,5 |
| K2O | 0,2 - 4,5 |
| CeO2 | 0,2 - 3,5 |
| B2O3 | 0,0 - 3,5 |
| Na2O | 0,0 - 3,5 |
| Tb4O7 | 0,0 - 2,0 |

| | |
|---|---|
| sowie 0,0 bis 4,0 von zumindest einem Additiv. | |

Bevorzugterweise besteht die Galsschmelze aus einer Zusammensetzung in Gew.-%:

| | |
|---|---|
| SiO2 | 52,0 - 66,0 |
| Li2O | 12,0 - 22,5 |
| ZrO2 | 7,5 - 13,0 |
| P2O5 | 2,0 - 8,5 |
| Al2O3 | 0,3 - 7,0 |
| K2O | 0,3 - 4,0 |
| CeO2 | 0,3 - 3,5 |
| B2O3 | 0,0 - 3,0 |
| Na2O | 0,0 - 3,0 |
| Tb4O7 | 0,0 - 2,0 |

| | |
|---|---|
| sowie 0,0 bis 4,0 von zumindest einem Additiv. | |

Besonders hervorzuheben ist eine Zusammensetzung der Glasschmelze mit einer Zusammensetzung in Gew.-%:

| | |
|---|---|
| SiO2 | 55,0 - 63,0 |
| Li2O | 12,5 - 21,5 |
| ZrO2 | 8,0 - 12,0 |
| P2O5 | 2,5 - 8,0 |
| Al2O3 | 0,4 - 6,5 |
| K2O | 0,4 - 4,0 |
| CeO2 | 0,5 - 3,0 |
| B2O3 | 0,0 - 3,0 |
| Na2O | 0,0 - 3,0 |
| Tb4O7 | 0,0 - 2,0 |

| | |
|---|---|
| sowie 0,0 bis 4,0 von zumindest einem Additiv. | |

Bevorzugterweise ist vorgesehen, dass die Glasschmelze eine Zusammensetzung in Gew.-% aufweist:

| | |
|---|---|
| SiO2 | 58,0 - 60,0 |
| Li2O | 13,5 - 20,5 |
| ZrO2 | 8,5 - 11,5 |
| P2O5 | 3,0 - 7,5 |
| Al2O3 | 0,5 - 6,0 |
| K2O | 0,5 - 3,5 |
| CeO2 | 0,5 - 2,5 |
| B2O3 | 0,0 - 3,0 |
| Na2O | 0,0 - 3,0 |
| Tb4O7 | 0,0 - 1,5 |

| | |
|---|---|
| sowie 0,0 bis 4,0 von zumindest einem Additiv. | |

Das zumindest eine Additiv ist zumindest ein Additiv aus der Gruppe Farbpigment, Fluoreszenzmittel. Insbesondere ist vorgesehen, dass das Additiv zumindest ein Oxid aus der Gruppe BaO, CaO, MgO, MnO, Er2O3, Gd2O3, Pr6O11, Sm2O3, TiO2, V2O5, Y203 ist oder ein solches Oxid enthält.

Das entsprechende Gemisch von Ausgangsstoffen, z. B. in Form von Oxiden und Carbonaten, wird sodann in einem geeigneten Tiegel aus feuerfestem Material oder Edelmetalllegierung bei einer Temperatur zwischen 1350 °C und 1600 °C über einen Zeitraum zwischen 1 h bis 10 h, insbesondere über einen Zeitraum von 4 h bis 7 h bei einer Temperatur von 1540 °C geschmolzen. Gleichzeitig oder anschließend erfolgt ein Homogenisieren, z. B. durch Rühren. Das so hergestellte flüssige Glas wird sodann einer vorzugsweise in Schwingung versetzten Düse zugeführt, die selbst auf eine Temperatur vorzugsweise im Bereich zwischen 1250 °C und 1450°C, insbesondere bei 1310 °C eingestellt ist. Die Düse kann einen Durchmesser zwischen 1 mm und 2 mm aufweisen. Die Schwingungsfrequenz der Düse kann zwischen 40 Hz und 60 Hz, insbesondere im Bereich von 50 Hz liegen. Das flüssige Glas wird sodann in einem geeigneten Medium wie Flüssigkeit, wie Wasser oder Hochtemperaturwatte abgeschreckt. Die so hergestellte abgeschreckte Glasfritte wird anschließend getrocknet. Sodann erfolgt ein Mahlen z. B. in einer Kugelmühle. Es erfolgt ein Sieben, wobei ein Sieb mit einer Maschenweite zwischen 50 µm und 500 µm benutzt werden kann. Nach Bedarf erfolgt ein weiteres Mahlen, z. B. durch eine Strahlenmühle (Jet mill) oder eine Attritionsmühle.

Aus dem so hergestellten Glas- bzw. Glaspartikelpulver werden insbesondere diejenigen ausgewählt, die einer Korngrößenverteilung d₉₀ ≤ 80 µm, insbesondere 10 µm ≤ d₅₀ ≤ 60 µm entsprechen. d₉₀ bzw. d₅₀ bedeutet, dass 90 % bzw. 50 % der vorhandenen Partikel einen Durchmesser besitzen, der kleiner als der angegebene Wert ist bzw. in dem Bereich liegen.

Damit der Rohling problemlos bearbeitet werden kann, ohne dass das aus dem Rohling hergestellte Formteil beim Durchsintern instabil ist, wird entweder die nach dem Schmelzen erhaltene Fritte oder das vor- bzw. endgemahlene Pulver einem Kristallisationsschritt unterzogen. Dabei wird in einem ersten Wärmebehandlungsschritt die Fritte bzw. das Pulver einer Temperatur T₁ zwischen 500 °C und 750 °C über eine Zeit t₁ zwischen 5 min und 120 min unterworfen. Der erste Wärmebehandlungsschritt kann auch zweistufig erfolgen, d. h. erster Wärmebehandlungsschritt 640 °C vorzugsweise 660 °C für 60 min und 750 °C für 40 min.

Bevorzugterweise schließt sich sodann eine weitere Wärmebehandlung in Form von Tempern an, wobei die zu wählende Temperatur T₃ zwischen 750 °C und 900 °C liegen sollte. Der Temperschritt wird über einen Zeitraum t₃ insbesondere zwischen 5 min und 30 min durchgeführt.

Sodann werden die Glaskeramikpartikel verpresst, wobei in Abhängigkeit von der herzustellenden Geometrie geeignete Pressverfahren, insbesondere ein axiales oder isostatisches Pressen oder Kombinationen dieser zur Anwendung gelangen. Das Verdichten erfolgt dabei in einem Umfang, dass die Dichte des Rohlings 30 % bis 60 % der theoretischen Dichte des Rohlingmaterials von etwa 2,64 g/cm³ entspricht. Insbesondere sollte der Rohling eine Dichte von in etwa 50 % der theoretischen Dichte aufweisen.

Zum Verpressen des Glaskeramikpulvers wird dieses vorzugsweise einem Druck zwischen 50 MPa und 400 MPA, insbesondere zwischen 100 MPa und 200 MPa ausgesetzt.

Die einzige Figur zeigt beispielhaft einen Verlauf des Drucks über die Zeit beim Verpressen eines Rohlings. In einer ersten Phase P1 wird der Druck ausgehend von 0 mit einem Druckaufbau von beispielsweise 15 MPa/sec auf einen Druck von beispielsweise 30 MPa erhöht. In einer zweiten Phase P2 wird der Druck von 30 MPa mit einem Druckaufbau von 100 MPa/sec auf einen Druck von ca. 200 MPa erhöht. In einer dritten Phase P3 wird der Druck auf einen Wert von ca. 200 MPa für eine Haltezeit von ca. 10 sec konstant gehalten. In einer vierten Phase erfolgt vorzugsweise ein zweistufiger Druckabbau, wobei in einer Phase P4a der Druck von ca. 200 MPa auf ca. 20 MPa mit einem Druckabbau von 40 MPa/sec und in einer Phase P4b der Druck von 20 MPa auf 0 MPa Überdruck mit einem Druckabbau von ca. 10 MPa/sec beaufschlagt wird.

Nach dem Verpressen erfolgt ein mechanisches Bearbeiten durch Fräsen, wobei zunächst eine Grobbearbeitung und sodann eine Feinbearbeitung durchgeführt werden kann. Das Bearbeiten kann ohne Kühlung erfolgen. Eine trockene Bearbeitung ist möglich.

Bei der Grobbearbeitung sollten nachstehende Fräsparameter beachtet werden:

| | |
|---|---|
| Fräserdurchmesser: | 2 bis 5 mm, besonders 2 bis 3 mm |
| Vorschub: | 500 bis 4000 mm/min, besonders 2000 bis 3000 mm/min |
| Seitliche Zustellung ae: | 0,2 bis 3 mm, besonders 1 mm bis 2 mm |
| Tiefenzustellung ap: | 0,1 bis 2 mm, besonders 0,5 mm bis 1 mm |
| Fräserdrehzahl: | 10.000 bis 50.000 1/min, besonders 10.000 bis 20.000 1/min. |

Das Fräswerkzeug sollte insbesondere ein Hartmetallfräser sein.

Fräsparameter für die Feinbearbeitung sollten sein:

| | |
|---|---|
| Fräserdurchmesser: | 0,3 bis 1,5 mm, besonders 0,5 bis 1,0 mm |
| Vorschub: | 300 bis 2000 mm/min, besonders 800 bis 1500 mm/min |
| Seitliche Zustellung ae: | 0,2 bis 0,6 mm, besonders 0,1 mm bis 0,2 mm |
| Tiefenzustellung ap: | 0,05 bis 0,3 mm, besonders 0,1 mm bis 0,15 mm |
| Fräserdrehzahl: | 20.000 bis 60.000 1/min, besonders 25.000 bis 35.000 1/min. |

Das Fräswerkzeug sollte insbesondere ein Hartmetallfräser sein.

Bevorzugterweise wird ein Kugelradiusfräser aus Hartmetall, der mit Titannitrid beschichtet sein kann, verwendet. Hierbei sind nachstehende Schneidenwinkel zu bevorzugen:

| | |
|---|---|
| Spanwinkel: | 0° bis -13°, besonders -9° bis -11° |
| Freiwinkel: | 0° bis 15°, besonders 11° bis 13° |
| Keilwinkel: | ergibt sich 90° minus Freiwinkel minus Spanwinkel. |

Aufgrund der Dichte des Rohlings und des kristallinen Anteils können dentale Formteile mit filigranen Rändern problemlos hergestellt werden. Bei Kronen hat sich gezeigt, dass sich stabile auslaufende Randdicken zwischen 0,05 mm und 0,4 mm ergeben.

Nach dem Herausarbeiten ist das aus dem Rohling hergestellte Formteil als Vorformteil zu bezeichnen, da dieses gegenüber dem dichtgesinterten dentalen Formteil entsprechend dem Schwund des Rohlingmaterials ein Übermaß aufweist. In Abhängigkeit von der Dichte des Rohlings wird das Übermaß berechnet, um einen hochpräzisen Zahnersatz als Endprodukt nach dem Dichtsintern zur Verfügung zu stellen.

Das Durch- bzw. Dichtsintern erfolgt bei einer Temperatur T₂ zwischen 800 °C und 1050°C über eine Haltezeit t₂ zwischen 5 min und 60 min. Haltezeit bedeutet dabei, dass der Rohling bei der gewünschten Endsintertemperatur auf dieser Temperatur gehalten wird.

Beim Durchsintern wird das Vorformteil auf eine feuerfeste Unterlage wie Brennwatte oder auf zunderfreie Metallschichten angeordnet. Stützstrukturen sind nicht erforderlich, da aufgrund der vorhergehenden Kristallisation des Ausgangspulvers die Formstabilität gesichert wird.

Aus nachstehenden Ausführungsbeispielen ergeben sich weitere die Erfindung kennzeichnenden Merkmale, wobei die angegebenen Parameter für sich und nicht zwingend in Kombination von besonderer Bedeutung sind:

### 1. Herstellung eines scheibenförmigen Rohlings

Eine Menge von 230g vorkristallisiertem, also Lithiumsilikatkristall enthaltenden Glaskeramikpulver der Zusammensetzung in Gew.-%:

| | |
|---|---|
| SiO2 | 58 - 60 |
| Li2O | 13,5 - 20,5 |
| ZrO2 | 8,5 - 11,5 |
| P2O5 | 3,0 - 7,5 |
| Al203 | 0,5 - 6,0 |
| K2O | 0,5 - 3,5 |
| CeO2 | 0,5 - 2,5 |
| B2O3 | 0 - 3 |
| Na2O | 0 - 3 |
| Tb4O7 | 0 - 1,5 |

| | |
|---|---|
| sowie 0 bis 4 von zumindest einem Additiv, | |

mit einer Korngrößenverteilung von d₅₀ = 18,7 µm, werden mit einem Druck von 50 MPa mittels eines Werkzeugs mit einem Durchmesser von 105 mm mit einer hydraulischen Presse vorverdichtet. Anschließend wird der Pressling in einen beschichteten PE-Beutel gegeben, evakuiert und wasserdicht eingeschweißt. In einer Wasser/Öl-Emulsion wird der Pressling bei 290 MPa für 10 sec isostatisch nachverdichtet. Nach dem Auspacken erfolgen eine Temperaturbehandlung und einen leichtes Ansintern bei 650 °C. Die Rohlingsdichte liegt bei 1,88 g/cm³.

Die endgültige Rohlingsgeometrie wird durch Überdrehen auf einen Außendurchmesser auf 98,5 mm hergestellt. Zur Aufnahme in eine Fräsmaschine wird an beiden Stirnseiten je ein Absatz gedreht.

In die eine Kreisgeometrie aufweisende Rohlingsfläche werden zahntechnische Formteile mit entsprechendem Sinteraufmaß genestet. Bei Kronen als Formteil weisen diese einen sehr guten feinen Kronenrand und eine hervorragende Fräsoberfläche auf.

Das Sintern erfolgt in einem Dental-Aufbrennofen auf Al₂O₃-Brennwatte mit einem gestuften Sinterprogramm über eine Gesamtzeit von 60 min. Gestuftes Sinterprogramm bedeutet, dass bei mindestens zwei verschiedenen Temperaturen Haltezeiten vorgesehen sind, für deren Dauer die jeweilige Temperatur konstant gehalten wird. Die maximale Sintertemperatur betrug 950 °C, die über 10 min gehalten wird. Die anschließende Beurteilung der Kronen ergab ein ästhetisches Erscheinungsbild mit einer guten zahntechnischen Passung.

### 2. Herstellung eines quaderförmigen Rohlings

Eine Menge von 9,6 g vorkristallisiertem Glaskeramikpulver der Zusammensetzung in Gew.-%.:

| | |
|---|---|
| SiO2 | 58 - 60 |
| Li2O | 13,5 - 20,5 |
| ZrO2 | 8,5 - 11,5 |
| P2O5 | 3,0 - 7,5 |
| Al2O3 | 0,5 - 6,0 |
| K2O | 0,5 - 3,5 |
| CeO2 | 0,5 - 2,5 |
| B2O3 | 0 - 3 |
| Na2O | 0 - 3 |
| Tb4O7 | 0 - 1,5 |

| | |
|---|---|
| sowie 0 bis 4 von zumindest einem Additiv, | |

mit einer Korngrößenverteilung von d₅₀ = 21,3 µm wird bei stetig ansteigendem Druck bis auf 120 MPa mit einer hydraulischen Presse in einer Hartmetallpressform axial verdichtet und unter einer geeigneten Auflast von vorzugsweise 5 MPa wieder entformt. Der erhaltene Pressling weist eine Abmessung von 20,2 x 19,1 x 15,9 mm und eine Dichte von 1,56 g/cm³ auf. Anschließend wird der Pressling in einem Elevatorofen einer zweistufigen Temperaturbehandlung bei 630 °C und 700 °C unterzogen. Die Rohlingsdichte stieg nach der Temperaturbehandlung auf 1,75 g/cm³.

Für die Aufnahme in eine Bearbeitungsmaschine wurde an die Schmalseite des Rohlings ein pilzförmiger Adapter geklebt. Das Ausarbeiten der um den Sinterschrumpf vergrößerten zahntechnischen Krone erfolgte über eine spezielle Speed-Fräsbearbeitung bei deutlich verkürzter Fräszeit mit einem Fräsvorschub von bis zu 2000 mm/min. Damit konnte die Fräszeit gegenüber einem nach Beispiel 1 hergestellten Teil deutlich verkürzt werden. Die Krone wies eine glatte Außenseite auf und der Kronenrand war frei von Ausbrüchen. Das Sintern erfolgte auf Al₂O₃-Brennwatte in einem Dental-Aufbrennofen mit einem gestuften Zyklus bei einer Gesamtzeit von 65 min und einer Sintermaximaltemperatur von 950 °C für 10 min. Eine anschließende Beurteilung der Krone ergab eine ästhetische Farbe mit einer guten zahntechnischen Passung.

### 3. Herstellung eines stabförmigen Rohlings

Eine Menge von 210 g vorkristallisiertem Glaskeramikpulver der Zusammensetzung in Gew.-%:

| | |
|---|---|
| SiO2 | 58 - 60 |
| Li2O | 13,5 - 20,5 |
| ZrO2 | 8,5 - 11,5 |
| P2O5 | 3,0 - 7,5 |
| Al2O3 | 0,5 - 6,0 |
| K2O | 0,5 - 3,5 |
| CeO2 | 0,5 - 2,5 |
| B2O3 | 0 - 3 |
| Na2O | 0 - 3 |
| Tb4O7 | 0 - 1,5 |

| | |
|---|---|
| sowie 0 bis 4 von zumindest einem Additiv, | |

mit einer Korngrößenverteilung von d₅₀ = 19,1 µm wird mit einer Wet-Bag-Presse bei einem quasi isostatischen Druck von 195 MPa in einem schlauchförmigen Polyurethan-Werkzeug verdichtet. Nach dem Entformen erfolgt eine Temperaturbehandlung für die zusätzliche Kristallisation bei 620 °C und dem Vorsintern bei 680 °C. Die endgültige Rohlingsgeometrie wird durch Überdrehen auf einen Außendurchmesser von 25 mm und eine Länge von 198 mm hergestellt. Die Rohlingsdichte beträgt 1,81 g/cm³.

Von den stangenförmigen Glaskeramikrohlingen werden stirnseitig zahntechnische Kronen mit entsprechendem Sinteraufmaß gefräst. Die Kronen weisen einen schmalen ausbruchsfreien Kronenrand und eine gute Fräsoberfläche auf. Das Sintern erfolgt in kleinen Kammeröfen auf Trays mit Al₂O₃-Brennwatte. Zur Anwendung kommt ein Sinterprogramm mit einer Gesamtzykluszeit von 45 min. Maximale Sintertemperaturbehandlung 980 °C. Bei dieser Temperatur wurde der Rohling für 5 min gehalten. Die fertigen Kronen zeigen ein ästhetisches Erscheinungsbild mit einer guten zahntechnischen Passung.

## Patentansprüche

1. Rohling zur Herstellung eines dentalen Formteils wie Inlay, Onlay, Krone oder Brücke, wobei der Rohling einen Anteil von mehr als 10 Vol.-% an Lithiumsilikatkristallen enthält,
**dadurch gekennzeichnet ,**
**dass** der Rohling aus einer Glaskeramik mit einer Dichte zwischen 30 % und 60 % der theoretischen Dichte und aus Glaskeramikpulverpartikeln einer Korngrößenverteilung d₉₀ ≤ 80 µm besteht, wobei der Anteil an Lithiumsilikatkristallen 10 Vol.-% bis 90 Vol.-% beträgt.

2. Rohling nach Anspruch 1,
**dadurch gekennzeichnet ,**
**dass** die Glaskeramikpulverpartikel eine Korngrößenverteilung d₅₀ ≤ 25 film aufweisen.

3. Rohling nach Anspruch 1 oder 2,
**dadurch gekennzeichnet ,**
**dass** der Rohling eine offene Porosität zwischen 5 Vol.-% und 60 Vol.-%, insbesondere zwischen 20 Vol.-% und 50 Vol.-% aufweist.

4. Rohling nach zumindest einem der vorhergehenden Ansprüche,
**dadurch gekennzeichnet ,**
**dass** der Rohling eine Scheiben-, Quader- oder Stabgeometrie aufweist, wobei insbesondere diametral zum Schwerpunkt verlaufende Mittel von der Umfangsfläche des Rohlings zum Anordnen in einer Fräsmaschine ausgehen.

5. Rohling nach zumindest einem der vorhergehenden Ansprüche,
**dadurch gekennzeichnet,**
**dass** die Glaskeramikpulverpartikel eine Zusammensetzung in Gew.-% aufweisen:
| | |
|---|---|
| SiO2 | 46,0 - 72,0 |
| Li2O | 10,0 - 25,0 |
| ZrO2 | 6,5 - 14,0 |
| P2O5 | 1,0 - 10,0 |
| Al2O3 | 0,1 - 8,0 |
| K2O | 0,1 - 5,0 |
| CeO2 | 0,1 - 4,0 |
| B2O3 | 0,0 - 4,0 |
| Na2O | 0,0 - 4,0 |
| Tb4O7 | 0,0 - 2,5 |
| | |
|---|---|
| sowie 0,0 bis 4,0 von zumindest einem Additiv. | |

6. Rohling nach zumindest einem der vorhergehenden Ansprüche,
**dadurch gekennzeichnet,**
**dass** die Glaskeramikpulverpartikel eine Zusammensetzung in Gew.-% aufweisen:
| | |
|---|---|
| SiO2 | 49,0 - 69,0 |
| Li2O | 11,5 - 24,0 |
| ZrO2 | 7,0 - 13,5 |
| P2O5 | 1,5 - 9,0 |
| Al2O3 | 0,2 - 7,5 |
| K2O | 0,2 - 4,5 |
| CeO2 | 0,2 - 3,5 |
| B2O3 | 0,0 - 3,5 |
| Na2O | 0,0 - 3,5 |
| Tb4O7 | 0,0 - 2,0 |
| | |
|---|---|
| sowie 0,0 bis 4,0 von zumindest einem Additiv. | |

7. Rohling nach zumindest einem der vorhergehenden Ansprüche,
**dadurch gekennzeichnet,**
**dass** die Glaskeramikpulverpartikel eine Zusammensetzung in Gew.-% aufweisen:
| | |
|---|---|
| SiO2 | 52,0 - 66,0 |
| Li2O | 12,0 - 22,5 |
| ZiO2 | 7,5 - 13,0 |
| P2O5 | 2,0 - 8,5 |
| Al2O3 | 0,3 - 7,0 |
| K2O | 0,3 - 4,0 |
| CeO2 | 0,3 - 3,5 |
| B2O3 | 0,0 - 3,0 |
| Na2O | 0,0 - 3,0 |
| Tb4O7 | 0,0 - 2,0 |
| | |
|---|---|
| sowie 0,0 bis 4,0 von zumindest einem Additiv. | |

8. Rohling nach zumindest einem der vorhergehenden Ansprüche,
**dadurch gekennzeichnet,**
**dass** die Glaskeramikpulverpartikel eine Zusammensetzung in Gew.-% aufweisen:
| | |
|---|---|
| SiO2 | 55,0 - 63,0 |
| Li2O | 12,5 - 21,5 |
| ZrO2 | 8,0 - 12,0 |
| P2O5 | 2,5 - 8,0 |
| Al2O3 | 0,4 - 6,5 |
| K2O | 0,4 - 4,0 |
| CeO2 | 0,5 - 3,0 |
| B2O3 | 0,0 - 3,0 |
| Na2O | 0,0 - 3,0 |
| Tb4O7 | 0,0 - 2,0 |
| | |
|---|---|
| sowie 0,0 bis 4,0 von zumindest einem Additiv. | |

9. Rohling nach zumindest einem der vorhergehenden Ansprüche,
**dadurch gekennzeichnet ,**
**dass** die Glaskeramikpulverpartikel eine Zusammensetzung in Gew.-% aufweisen:
| | |
|---|---|
| SiO2 | 58 - 60 |
| Li2O | 13,5 - 20,5 |
| ZrO2 | 8,5 - 11,5 |
| P2O5 | 3,0 - 7,5 |
| Al2O3 | 0,5 - 6,0 |
| K2O | 0,5 - 3,5 |
| CeO2 | 0,5 - 2,5 |
| B2O3 | 0 - 3 |
| Na2O | 0 - 3 |
| Tb4O7 | 0 - 1,5 |
| sowie 0,0 bis 4,0 von zumindest einem Additiv. | |

10. Rohling nach zumindest einem der vorhergehenden Ansprüche,
**dadurch gekennzeichnet ,**
**dass** das Additiv zumindest ein Additiv aus der Gruppe Farbpigment, Fluoreszenzmittel ist, und/oder
**dass** das Additiv zumindest ein Oxid aus der Gruppe BaO, CaO, MgO, MnO, Er2O3, Gd2O3, Pr6O11, Sm2O3, TiO2, V2O5, Y203 ist.

11. Rohling nach zumindest einem der vorhergehenden Ansprüche,
**dadurch gekennzeichnet ,**
**dass** der Anteil der Lithiumsilikatkristalle in der Glaskeramik beträgt 40 Vol.-% bis 60 Vol.-%.

12. Monolithisches dentales Formteil unter Verwendung eines Rohlings nach zumindest einem der Ansprüche 1 bis 11.

13. Monolithisches dentales Formteil nach Anspruch 12,
**dadurch gekennzeichnet ,**
**dass** das dentale Formteile eine Krone ist oder umfasst und einen Kronenrand einer Dicke D_{R} mit 0,05 mm ≤ D_{R} ≤ 0,4 mm, insbesondere 0,1 mm ≤ D_{R} ≤ 0,2 mm aufweist.

14. Monolithisches dentales Formteil nach Anspruch 12 oder 13,
**dadurch gekennzeichnet ,**
**dass** das Formteil einen Wärmeausdehnungskoeffizienten WAK mit WAK ≤ 12.5 x 10⁻⁶ 1/K, insbesondere 9,5 x 10⁻⁶ 1/K ≤ WAK ≤ 11,5 x 10⁻⁶ 1/K aufweist.

15. Monolithisches dentales Formteil nach einem der Ansprüche 12 - 14, hergestellt mit einem Verfahren umfassend die Verfahrensschritte
- Herstellen einer Schmelze aus einer Schmelze der Zusammensetzung in Gew.-%:
| | |
|---|---|
| SiO2 | 46,0 - 72,0 |
| Li2O | 10,0 - 25,0 |
| ZrO2 | 6,5 - 14,0 |
| P2O5 | 1,0 - 10,0 |
| Al2O3 | 0,1 - 8,0 |
| K2O | 0,1 - 5,0 |
| CeO2 | 0,1 - 4,0 |
| B2O3 | 0,0 - 4,0 |
| Na2O | 0,0 - 4,0 |
| Tb4O7 | 0,0 - 2,5 |
| | |
|---|---|
| sowie 0,0 bis 4,0 von zumindest einem Additiv, | |
- Herstellen einer Glasfritte durch Verdüsen der Schmelze und Abschrecken in einem Medium,
- gegebenenfalls Erzeugen von Glaspulverpartikeln aus der Glasfritte mit einer Korngrößenverteilung d₉₀ ≤ 80 µm,
- Auskristallisieren von Lithiumsilikatkristallen mit einem Volumenanteil zwischen 10 % und 90 % durch eine erste Wärmebehandlung von entweder der Glasfritte oder den Glaspulverpartikeln in einem ersten Temperaturbereich bei einer Temperatur T₁ mit 500 °C ≤ T₁ ≤ 750 °C über eine Zeit t₁ mit 5 min ≤ t₁ ≤ 120 min,
- wobei dann, wenn die Glasfritte einer Wärmebehandlung unterzogen wird, aus der wärmebehandelten Glasfritte Herstellen von Glaskeramikpartikeln mit einer Korngrößenverteilung d₉₀ ≤ 80 µm erfolgt,
- Pressen der Glaskeramikpulverpartikel zu einem Rohling,
- Bearbeiten des Rohlings durch Fräsen zur Herstellung eines dem dentalen Formteil entsprechenden Vorformteils unter Berücksichtigung des Schwundverhaltens des Rohlings und
- Durchsintern des Vorformteils bei einer Temperatur T₂ mit 800 °C ≤ T₂ ≤ 1050 °C über eine Zeit t₂ mit 5 min ≤ t₂ ≤ 60 min.
